# EUROPÄISCHE PATENTSCHRIFT

(11) **EP 3 231 792 B1**
(45) Veröffentlichungstag und Bekanntmachung des Hinweises auf die Patenterteilung: **19.06.2019**
(21) Anmeldenummer: 17000622.5
(22) Anmeldetag: 11.04.2017
(51) Int. Cl.: C07D 231/12

(54) **VERFAHREN ZUR HERSTELLUNG EINES GEMISCHES AUS N-((3-METHYL-1H-PYRAZOL-1-YL)METHYL)ACETAMID UND N-((5-METHYL-1H-PYRAZOL-1-YL)METHYL)ACETAMID**
METHOD FOR THE PREPARATION OF A MIXTURE OF N-((3-METHYL-1H-PYRAZOLE-1-YL)METHYL)ACETAMIDE AND N-((5-METHYL-1H-PYRAZOLE-1-YL)METHYL)ACETAMIDE
PROCÉDÉ DE FABRICATION D'UN MELANGE DE N-((3-METHYL-1H-PYRAZOLE-1-YL)METHYL)ACETAMIDE ET N-((5-METHYL-1H-PYRAZOLE-1-YL)METHYL)ACETAMIDE

(30) Priorität: 11.04.2016 DE 102016106592
(43) Veröffentlichungstag der Anmeldung: 18.10.2017
(73) Patentinhaber: SKW STICKSTOFFWERKE PIESTERITZ GmbH, 06886 Lutherstadt Wittenberg (DE)
(72) Erfinder: Radics, Ute, 06901 Kemberg (DE); Niclas, Hans-Joachim, 12435 Berlin (DE); Schuster, Carola, 06886 Lutherstadt Wittenberg (DE)
(74) Vertreter: Grünecker Patent- und Rechtsanwälte PartG mbB

(56) Entgegenhaltungen:
- EP-B1- 1 663 997
- DE-A1-102011 120 098

## Beschreibung

Die vorliegende Erfindung betrifft ein Verfahren zur Herstellung von N-((3(5)-Methyl-1H-pyrazol-1-yl)methyl)acetamid gemäß den Patentansprüchen.

### Stand der Technik

Weltweit erfolgt die Düngung mit Stickstoff überwiegend mit Hilfe von Harnstoff oder auch Harnstoffmischungen mit anderen Düngerkomponenten wie beispielsweise Ammoniumsulfat.

Die Düngewirkung des Harnstoffs im Ackerboden beruht auf der hydrolytischen Umwandlung vom Harnstoff durch das ubiquitär im Boden vorhandene Enzym Urease zu Ammoniumionen und Hydrogencarbonat. Im biologisch aktiven Boden kann das Ammoniumion unter Einwirkung von Nitrosomas- und Nitrobacter-Bakterien über Nitrit sehr schnell zum Nitrat oxidiert werden.

Durch Nitrifikationsinhibitoren wird die mikrobielle Umwandlung von Ammoniumstickstoff zu Nitratstickstoff im Boden über einen gewissen Zeitraum gehemmt und dadurch eine verbesserte Stickstoffausnutzung durch die Pflanze bewirkt, da das Risiko der Nitratverlagerung deutlich gemindert ist. Gleichzeitig können Denitrifikationsverluste um über 50 % reduziert werden.

Die Anwendung von mit Nitrifikationsinhibitoren kombinierten Stickstoffdüngern ermöglicht auf Grund ihrer Wirkungsweise sowohl wirtschaftliche als auch ökologische Vorteilseffekte. Infolge der über einen Zeitraum von 4 bis 12 Wochen andauernden Hemmung der mikrobiellen Oxidation des gedüngten Ammoniumstickstoffs zu Nitrat liegt der Stickstoff vorwiegend in Form von Ammonium (für die Pflanzen verfügbar) vor und es werden N-Verluste durch

Nitratauswaschung und Denitrifikation reduziert. Die Verminderung des Verlustpotenzials erlaubt, verbunden mit gewissen Vorteilen einer ammoniumbetonten Pflanzenernährung, eine Reduzierung des N-Aufwandes bei gleichbleibend hohem Ertragsniveau sowie eine Zusammenlegung von Arbeitsgängen, was zu arbeitswirtschaftlichen Vorteilen führt.

Mit dem Einsatz von N-stabilisierten Düngern kann wesentlich zur Reduzierung von ökologisch unerwünschten N-Austrägen aus landwirtschaftlichen Systemen, zur Verringerung von Treibhausgas-Emissionen sowie zur Minderung der Stickstoffüberschüsse in der Landwirtschaft beigetragen werden. Dies sind eine umweltpolitische Forderungen, der die landwirtschaftliche Produktion zunehmend gerecht werden muss.

Nachweislich wirkt sich der Einsatz von mit Nitrifikationsinhibitoren versehenen Stickstoffdüngemitteln besonders vorteilhaft in Wasserschutzzonen aus, da durch die verzögerte Nitratbildung der Eintrag dieser N-Form in Gewässer erheblich reduziert werden kann.

Als wirksame Nitrifikationsinhibitoren sind eine große Zahl verschiedener Substanzen und Substanzgemische vorgeschlagen worden (siehe u. a. M.E. TRENKEL, Slow- and Controlleded-Release and Stabilized Fertilizers - An Option for Enhancing Nutrient Use Efficiency in Agriculture; International Fertilizer Industry Assoziation (ifa), Paris Oct. 2010).

Für den praktischen Einsatz als Nitrifikationsinhibitoren eignen sich jedoch aufgrund ihrer chemischen und physikalischen Eigenschaften nur wenige der vorgeschlagenen Substanzen. Entsprechend hergestellte sogenannte "stabilisierte Feststoffdünger" finden in der landwirtschaftlichen Praxis eine hohe Akzeptanz, so dass ein weltweiter Bedarf besteht.

In EP 166 3 997 B1 und DE 10 2011 120 098 A1 werden N-(1H-Azolyl-methyl)amide als Einkomponentenwirkstoffe offenbart, die auch bei geringen Aufwandmengen ausgezeichnete nitrifikationshemmende Eigenschaften zeigen und auch in Gegenwart von harnstoffhaltigen Düngemitteln hydrolysestabil sind ohne dass sie vorher in ihre Salze überführt werden müssen, um Wirkstoffverluste infolge Flüchtigkeit zu verhindern.

In diesen Patentschriften werden auch verschiedene Syntheseverfahren beschrieben, die man für die Laborsynthese von N-((3(5)-Methyl-1H-pyrazol-1-yl)methyl)acetamid (MPA) verwenden kann. In Bezug auf die Produktausbeute und den notwendigen Aufwand, der für die Aufarbeitung des Reaktionsgemisches betrieben werden muss, können diese jedoch noch keineswegs befriedigen, wie die folgende Tabelle 1 belegt.

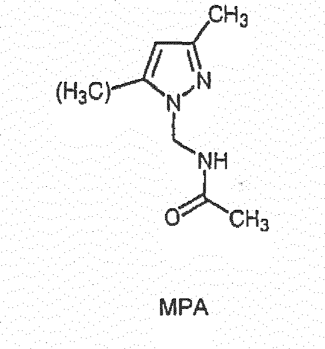

**Tabelle 1: Produktausbeute und Isomerenverhältnis für MPA, hergestellt nach EP 166 3 997 B1 und DE 10 2011 120 098 A1**

| Variante | Beschreibung | MPA-Ausbeute in % | Isomeren verhältnis 3:5 |
|---|---|---|---|
| (1) | 0,5 mol (IV); 075 mol (I); 10 Tr. H₂SO₄ 250 ml Toluol; 3 h 115 °C unter Abdestillieren des Wassers | 64 | 50:50 bis 87:13 |
| | Aufarbeitung: Säulenchromatographie | | |
| (2) | 0,5 mol (V); 0,5 mol (III); Reaktion in der Schmelze; 1 h 140 °C | 13 | 18:82 |
| | Schmelze mit Wasser behandelt | | |
| (3) | 0,2 mol (1); 0,2 mol (II); 0,2 mol (III); 5 h 140 °C | 46 | 59:41 |
| | Aufarbeitung mit MTBE und n-Heptan | | |

N-((3(5)-Methyl-1H-pyrazol-1-yl)methyl)acetamid (MPA) ist ein Isomerengemisch. Bei der Synthese fällt es in der Regel in einem Isomerenverhältnis von 2 Teilen 3-Methylverbindung und 1 Teil 5-Methylverbindung an. Die beiden Isomere unterscheiden sich in einigen physikochemischen Eigenschaften wie dem Schmelzpunkt und insbesondere in ihrer Löslichkeit. Die 3-Methylverbindung ist immer deutlich leichter löslich. Wird die Aufarbeitung des Reaktionsgemisches unter Verwendung von Lösungsmitteln durchgeführt, ist damit zu rechnen, dass das ursprüngliche Isomerenverhältnis verschoben wird. Dies wird experimentell mit den Ergebnissen aus Tabelle 1 belegt.

Es bestand die Aufgabe, ein Herstellungsverfahren für N-((3(S)-Methyl-1H-pyrazol-1-yl)methyl)acetamid (MPA) zu finden, das es gestattet die Zielverbindung durch eine unkomplizierte, ökonomisch sinnvolle Synthese und Reinigung in hohen Ausbeuten und guter Reinheit sowie mit gut reproduzierbarem Isomerenverhältnis im technischen Maßstab bereit zu stellen.

### Beschreibung der Erfindung

Diese Aufgabe wird erfindungsgemäß gelöst, in dem N-((3(5)-Methyl-1H-pyrazol-1-yl)methyl)acetamid (MPA) in einer Dreikomponentenreaktion aus den kommerziell verfügbare Rohstoffen 3-Methylpyrazol (I), Formaldehyd (II) und Acetamid (III) bei Temperaturen von mehr als 120 °C bis 160 °C, bevorzugt bei 140 bis 150 °C, ohne Lösungsmittel und mit einem Molverhältnis von 3-Methylpyrazol (I) zu Formaldehyd (II) und Acetamid (III) von 0,7 : 0,8 : 1 bis 1,5 : 2,0 : 1, insbesondere 0,7 : 0,9 : 1 bis 0,9 : 1,4 : 1 unter Wasserabscheidung synthetisiert wird. Die Reaktionszeit beträgt bevorzugt 3 bis 10, besonders bevorzugt 5 Stunden.

Die Reinigung erfolgt durch Abdestillieren der überschüssigen Ausgangsstoffe im Vakuum bei Temperaturen von oberhalb 120 ^C bis 150 °C. Durch diese Reinigungsmethode lässt sich ein reproduzierbares Isomerenverhältnis (3 : 5-Methyl-Isomeres) erzielen.

Werden 3-Methylpyrazol (I), Formaldehyd (II) und Acetamid (III) als kommerziell verfügbare Rohstoffe für die Herstellung von MPA gewählt sind drei Synthesevarianten möglich (siehe Gleichung 1 bis 3). So können zum einen Monohydroxymethylacetamid (IV) mit 3-Methylpyrazol (I) (GI. (2)) oder 1-Hydroxmethyl-3(5)-methylpyrazol (V) mit Acetamid (III) (GI. (3)) unter Wasserabspaltung zur Reaktion gebracht werden. Bei beiden Verfahren wird Formaldehyd zunächst mit nur einem weiteren Ausgangsstoff zu einem einheitlichen Zwischenprodukt (IV oder V) umgesetzt. Dies hat den Vorteil, dass bei der Finalreaktion zum MPA kein überschüssiger Formaldehyd aus dem Reaktionsprodukt entfernt werden muss. Bei der Variante nach Gleichung (1) erfolgt die MPA-Bildung ohne dass ein Zwischenprodukt isoliert wird. Ist die Umsetzung der Komponenten nicht vollständig sollte bei dieser Variante die Vielzahl und Menge an Nebenbestandteilen im Reaktionsprodukt am Größten sein.

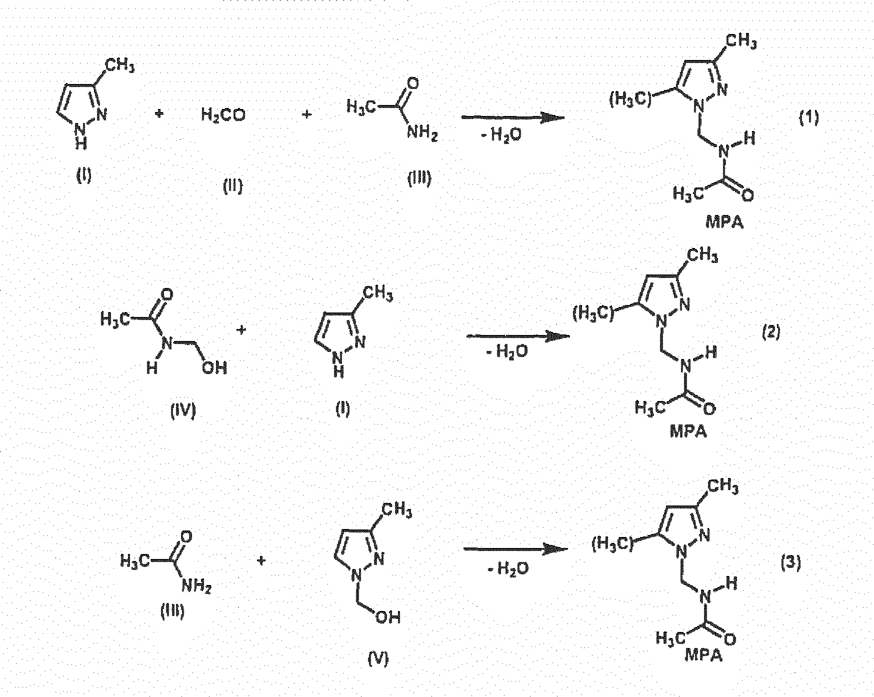

Es hat sich nun überraschenderweise gezeigt, dass entgegen dieser Annahme bei Durchführung der Dreikomponentenreaktion nach Gleichung (1) die Ausbeute an MPA > 90 % ist wenn 3-Methylpyrazol (I), Formaldehyd (II) (z. B. in Form von Paraformaldehyd) und Acetamid (III) bei Temperaturen von mehr als 120 °C bis 160 °C, bevorzugt bei 140 bis 150 °C, ohne Lösungsmittel und mit einem Molverhältnis von 3-Methylpyrazol (I) zu Formaldehyd (II) und Acetamid (III) von 0,7 : 0,8 : 1 bis 1,5 : 2,0 : 1, insbesondere 0,7 : 0,9 : 1 bis 0,9 : 1,4 : 1 unter Wasserabscheidung zur Reaktion gebracht werden. Die Reaktionszeit beträgt bevorzugt 3 bis 10, besonders bevorzugt 5 Stunden.

Die Reinigung erfolgt durch Abdestillieren der überschüssigen Ausgangsstoffe im Vakuum. Dazu wird bei Temperaturen von oberhalb 120 °C bis 150 °C, bevorzugt bei 145 °C destilliert wobei das Vakuum vorzugsweise schrittweise bis auf 20 bis 3 mbar, bevorzugt auf 10 bis 5 mbar abgesenkt wird. Durch diese Reinigungsmethode lässt sich ein reproduzierbares Isomerenverhältnis von 63/37 bis 73/27 (3 : 5-Methyl-lsomeres), bevorzugt 65/35 bis 70/30 erzielen.

Von entscheidender Bedeutung bei der Reaktion ist dabei, dass Paraformaldehyd (II) gegenüber 3-Methylpyrazol (I) im Überschuss eingesetzt wird.

Dieses Beispiel demonstriert den verbesserten Umsetzungsgrad bei der Synthese von MPA durch Einsatz von überschüssigem Paraformaldehyd.

| | | | | | | | |
|---|---|---|---|---|---|---|---|
| Acetamid (mol) | 1,125 | 1,0 | 1,15 | 1,125 | 1,3 | 1,3 | 1,04 |
| Paraformaldehyd (mol) | 1,1 | 1,0 | 1,3 | 1,25 | 1,25 | 1,0 | 0,8 |
| 3-Methylpyrazol (mol) | 1,0 | 1,0 | 1,0 | 1,0 | 1,0 | 1,0 | 1,0 |
| Verunreinigungen (HPLC) in % | 6,1 | 11,6 | 8,9 | 2,8 | 4,4 | 9,7 | 19,9 |

| | | | | | | | |
|---|---|---|---|---|---|---|---|
| Reaktionsbedingungen: 5 h 140 bis 150 °C unter Abdestillieren von Wasser | | | | | | | |

Bei der erfindungsgemäßen Produktreinigung tritt so gut wie keine Verschiebung des Isomerenverhältnisses auf.

Dieses Ergebnis war nicht vorauszusehen. Zum einen bewegen sich die Temperatur und das für den gewünschten Reinigungseffekt erforderliche Endvakuum in der Nähe des Siedepunktes von MPA, so dass es denkbar war, dass sich das später siedende Isomere von MPA im Finalprodukt anreichert und damit eine Verschiebung des Isomerenverhältnis eintritt. Zum anderen kann es beim längeren Erhitzen von MPA oberhalb des Schmelzpunktes zur Produktzersetzung kommen, so dass die Eignung der erfindungsgemäßen Reinigungsmethode nicht vorherzusehen war.

| | | | | | | | |
|---|---|---|---|---|---|---|---|
| T in °C | 140 | 145 | 145 | 145 | 155 | 155 | 160 |
| Enddruck in mbar | 5 | 3 | 6 | 7 | 5 | 7 | 5 |
| MPA-Ausbeute in % | 90,1 | 80,7 | 95,3 | 95,1 | 87,7 | 90,4 | 32,0 |
| Verunreinigungen (HPLC) in % | 3,4 | 3,0 | 3,1 | 2,8 | 2,85 | 3,3 | 11.1 |
| Isomeren-verhältnis 3:5 | 69:31 | 67:33 | 67:33 | 70:30 | 67:33 | 68:62 | 65:35 |

### Beispiele:

### Beispiel 1:

In einem 1-Liter Dreihalskolben werden 4 mol 3-Methylpyrazol vorgelegt. Unter Rühren werden 5,2 mol Acetamid und 5 mol Paraformaldehyd zugegeben. Das System wird mit Schutzgas (Ar, N₂) gespült und gut durchgerührt. Unter weiterer Schutzbegasung und Rühren wird in ca. 1 Stunde auf 150 °C erhitzt und diese Temperatur danach fünf Stunden lang gehalten. Dabei wird das entstehende Reaktionswasser abdestilliert.

Anschließend werden unter Rühren die überschüssigen Ausgangsstoffe durch schrittweise Absenkung des Vakuums bis auf 5 mbar abdestilliert. Belüftet wird mit Schutzgas.

Nach Beendigung der Reinigung lässt man das Produkt auf ca. 130 °C abkühlen und gießt dann die flüssige Schmelze in eine Edelstahlschale. Beim Abkühlen kristallisiert das MPA durch. Nach 48 Stunden kann das Produkt mechanisch zerkleinert und abgefüllt werden. Es werden 557 g MPA isoliert.
Ausbeute: 91 %

### Beispiel 2:

In einem 1-Liter Dreihalskolben werden 4 mol 3-Methylpyrazol vorgelegt. Unter Rühren werden 4,5 mol Acetamid und 5 mol Paraformaldehyd zugegeben. Das System wird mit Schutzgas (Ar, N₂) gespült und gut durchgerührt. Unter weiterer Schutzbegasung und Rühren wird in ca. 1 Stunde auf 145 °C erhitzt und diese Temperatur danach fünf Stunden lang gehalten. Dabei wird das entstehende Reaktionswasser abdestilliert.

Anschließend werden unter Rühren die überschüssigen Ausgangsstoffe durch schrittweise Absenkung des Vakuums bis auf 6 mbar abdestilliert. Belüftet wird mit Schutzgas.

Nach Beendigung der Reinigung lässt man das Produkt auf ca. 130 °C abkühlen und gießt dann die flüssige Schmelze in eine Edelstahlschale. Beim Abkühlen kristallisiert das MPA durch. Nach 48 Stunden kann das Produkt mechanisch zerkleinert und abgefüllt werden. Es werden 580 g MPA isoliert.
Ausbeute: 95 %

## Patentansprüche

1. Verfahren zur Herstellung von N-((3(5)-Methy)-1H-pyrazol-1-yl)methyl)acetamid (MPA), mit einer Dreikomponentenreaktion aus 3-Methylpyrazol (I), Formaldehyd (II) und Acetamid (III) bei Temperaturen von mehr als 120°C bis 160°C ohne Lösungsmittel, **dadurch gekennzeichnet, dass** die Ausgangsstoffe mit einem Molverhältnis von 3-Methylpyrazol (I) zu Formaldehyd (II) und Acetamid (III) von 0,7 : 0,8 : 1 bis 1,5 : 2,0 : 1, unter WasserabScheidung, zur Reaktion gebracht werden und
die Reinigung durch Abdestillieren der überschüssigen Ausgangsstoffe im Vakuum bei Temperaturen von oberhalb 120°C bis 150°C erfolgt.

2. Verfahren nach Anspruch 1, wobei die Dreikomponentenreaktion bei 140 bis 160°C durchgeführt wird.

3. Verfahren nach Anspruch 1, wobei das Molverhältnis von 3-Methylpyrazol (I) zu Formaldehyd (II) und Acetamid (III) 0,7 : 0,9 : 1 bis 0,9 : 1,4 : 1 beträgt.

4. Verfahren nach Anspruch 1 oder 2, wobei die Reaktionszeit 3 bis 10 Stunden beträgt.

5. Verfahren nach Anspruch 4, wobei die Reaktionszeit 5 Stunden beträgt.

6. Verfahren nach einem der Ansprüche 1, 2 oder 4, wobei das Vakuum schrittweise bis auf 20 bis 3 mbar abgesenkt wird.

7. Verfahren nach Anspruch 6, wobei das Vakuum schrittweise bis auf 10 bis 5 mbar abgesenkt wird.

8. Verfahren nach Anspruch 1 oder 2, wobei die Temperatur der Vakuumdestillation 140 bis 150°C beträgt.

9. Verfahren nach Anspruch 8, wobei die Temperatur der Vakuumdestillation 145°C beträgt.

10. Verfahren nach Anspruch 6 oder 7, wobei die Temperatur der Vakuumdestillation 140 bis 150°C beträgt.

11. Verfahren nach Anspruch 10, wobei die Temperatur der Vakuumdestillation 145°C beträgt.

12. Verfahren nach einem der Ansprüche 10 oder 11, wobei ein Isomerenverhältnis (3: 5-Methyl-Isomeres) von 65/35 bis 70/30 erzielt wird.

## Claims

1. A method for producing N-((3(5)-methyl-1H-pyrazol-1-yl)methyl)acetamide (MPA) using a three-component reaction of 3-methylpyrazol (I), formaldehyde (II) and acetamide (III) at temperatures above 120°C to 160°C without a solvent, **characterized in that** the precursors are reacted at a mol ratio of 3-methylpyrazol (I) to formaldehyde (II) to acetamide (III) of 0.7 : 0.8 : 1 to 1.5 : 2.0 : 1 while undergoing water separation and
the purification is carried out by distilling off the excess precursors in a vacuum at temperatures of above 120 °C to 150 °C.

2. The method according to claim 1, wherein the three-component reaction is carried out at 140 to 160 °C.

3. The method according to claim 1, wherein the mol ratio of 3-methylpyrazol (I) to formaldehyde (II) to acetamide (III) is 0.7 : 0.9 : 1 to 0.9 : 1.4 : 1.

4. The method according to claim 1 or 2, wherein the reaction time is 3 to 10 hours.

5. The method according to claim 4, wherein the reaction time is 5 hours.

6. The method according to one of the claims 1, 2 or 4, wherein the vacuum is gradually reduced to 20 to 3 mbar.

7. The method according to claim 6, wherein the vacuum is gradually reduced to 10 to 5 mbar.

8. The method according to claim 1 or 2, wherein the temperature of the vacuum distillation is 140 to 150 °C.

9. The method according to claim 8, wherein the temperature of the vacuum distillation is 145 °C.

10. The method according to claim 6 or 7, wherein the temperature of the vacuum distillation is 140 to 150 °C.

11. The method according to claim 10, wherein the temperature of the vacuum distillation is 145 °C.

12. The method according to one of the claims 10 or 11, wherein an isomeric ratio (3 : 5-methyl-isomeres) of 65/35 to 70/30 is achieved.

## Revendications

1. Procédé pour la production de N-((3(5)-méthyl-1H-pyrazole-1-yl)méthyl)acétamide (MPA) avec une réaction à trois composants mettant enjeu le 3-méthylpyrazole (I), le formaldéhyde (II) et l'acétamide (III) à des températures de plus de 120 °C à 160 °C sans solvant, **caractérisé en ce que** les précurseurs sont mis en réaction selon un rapport molaire du 3-méthylpyrazole (I) au formaldéhyde (II) à l'acétamide (III) de 0,7 : 0,8 : 1 à 1,5 : 2,0 : 1, avec séparation de l'eau et
la purification est réalisée en séparant par distillation les précurseurs excédentaires sous vide à des températures de plus de 120°C à 150 °C.

2. Procédé selon la revendication 1, où la réaction à trois composants est réalisée à 140 à 160 °C.

3. Procédé selon la revendication 1, où le rapport molaire du 3-méthylpyrazole (I) au formaldéhyde (II) et à l'acétamide (III) est de 0,7 : 0,9 : 1 à 0,9 : 1,4 : 1.

4. Procédé selon la revendication 1 ou 2, où le temps de réaction est de 3 à 10 heures.

5. Procédé selon la revendication 4, où le temps de réaction est de 5 heures.

6. Procédé selon l'une des revendications 1, 2 ou 4, où le vide est graduellement réduit à 20 à 3 mbar.

7. Procédé selon la revendication 6, où le vide est graduellement réduit à 10 à 5 mbar.

8. Procédé selon la revendication 1 ou 2, où la température de la distillation sous vide est de 140 à 150°C.

9. Procédé selon la revendication 8, où la température de la distillation sous vide est de 145 °C.

10. Procédé selon la revendication 6 ou 7, où la température de la distillation sous vide est de 140 à 150°C.

11. Procédé selon la revendication 10, où la température de la distillation sous vide est de 145 °C.

12. Procédé selon l'une des revendications 10 ou 11, où un rapport isomérique (isomères de 3 : 5-méthyle) de 65/35 à 70/30 est obtenu.
